(19) Europäisches Patentamt

European Patent Office

Office européen des brevets

(11) Veröffentlichungsnummer: **0 618 840 B1**

(12) **EUROPÄISCHE PATENTSCHRIFT**

(45) Veröffentlichungstag der Patentschrift: **30.08.95**

(21) Anmeldenummer: **92924628.8**

(22) Anmeldetag: **30.11.92**

(86) Internationale Anmeldenummer:
**PCT/EP92/02766**

(87) Internationale Veröffentlichungsnummer:
**WO 93/11865 (24.06.93 93/15)**

(51) Int. Cl.⁶: **B01F 17/00**, B01F 17/38,
B01F 17/06, B01F 17/36,
//B01F17/42,A61K7/00

(54) **VERFAHREN ZUR HERSTELLUNG VON ÖL-IN-WASSER-EMULSIONEN.**

(30) Priorität: **09.12.91 DE 4140562**

(43) Veröffentlichungstag der Anmeldung:
**12.10.94 Patentblatt 94/41**

(45) Bekanntmachung des Hinweises auf die
Patenterteilung:
**30.08.95 Patentblatt 95/35**

(84) Benannte Vertragsstaaten:
**BE DE ES FR GB IT NL SE**

(56) Entgegenhaltungen:
EP-A- 0 214 568
EP-A- 0 345 586
WO-A-89/11907
WO-A-91/15184
FR-A- 2 304 392

**PATENT ABSTRACTS OF JAPAN vol. 4, no.
89 (C-16)(571) 25. Juni 1980 & JP,A,55 053
210 ( SHISEIDO K.K. ) 18 April 1980**

(73) Patentinhaber: **Henkel Kommanditgesellschaft
auf Aktien**

**D-40191 Düsseldorf (DE)**

(72) Erfinder: **FÖRSTER, Thomas
Adalbert-Stifter-Stra e 15
D-4006 Erkrath (DE)**
Erfinder: **CLAAS, Marcus
Schützenstra e 70 b
D-4010 Hilden (DE)**
Erfinder: **WACHTER, Rolf
Clausthal-Zellerfelder-Stra e 48
D-4000 Düsseldorf (DE)**
Erfinder: **TESMANN, Holger
Vennstra e 61
D-4000 Düsseldorf (DE)**

**Beschreibung**

Die Erfindung betrifft ein Verfahren zur Herstellung von Öl-in-Wasser-Emulsionen auf Basis von Ölmischungen mit einem hohen Anteil an polaren Ölkomponenten unter Bedingungen, die zu feindispersen und langzeitstabilen Emulsionen führen.

Es ist bekannt, daß Öl-in-Wasser-Emulsionen, fortan O/W-Emulsionen genannt, die mit nichtionischen Emulgatoren hergestellt und stabilisiert sind, beim Erwärmen eine Phaseninversion erleiden. Unter diesem Vorgang der Phaseninversion ist zu verstehen, daß bei höheren Temperaturen die äußere, wäßrige Phase zur inneren Phase wird. Dieser Vorgang ist in der Regel reversibel, das heißt, daß sich beim Abkühlen wieder der ursprüngliche Emulsionstyp zurückbildet. Es ist auch bekannt, daß die Lage der Phaseninversionstemperatur von vielen Faktoren abhängt, zum Beispiel von der Art und dem Phasenvolumen der Ölkomponente, von der Hydrophilie und der Struktur des Emulgators oder der Zusammensetzung des Emulgatorsystems, vergleiche zum Beispiel K. Shinoda und H. Kunieda in Encyclopedia of Emulsion Technology, Volume I, P. Becher (Hrsg.), Verlag Marcel Decker, New York 1983, S. 337 ff. Es ist auch bekannt, daß O/W-Emulsionen, die bei oder wenig oberhalb der Phaseninversionstemperatur hergestellt werden, besonders feindispers sind und sich durch Langzeit-Stabilität auszeichnen. Demgegenüber sind solche Emulsionen, die unterhalb der Phaseninversionstemperatur hergestellt werden, weniger feinteilig, vergleiche S. Friberg, C. Solans, J. Colloid Interface Science 1978 [66] 367 f.

F. Schambil, F. Jost und M. J. Schwuger berichten in "Progress and Colloid and Polymer Science" 1987 [73] 37 über die Eigenschaften kosmetischer Emulsionen, die Fettalkohole und Fettalkoholpolyglykolether enthalten. Dabei beschreiben sie, daß Emulsionen, die oberhalb der Phaseninversionstemperatur hergestellt wurden, eine niedrige Viskosität und eine hohe Lagerstabilität aufweisen.

In den genannten Druckschriften wurden jedoch nur Emulsionen untersucht, deren Ölphase ganz oder überwiegend aus unpolaren Kohlenwasserstoffen besteht. Demgegenüber verhalten sich entsprechende Emulsionen, deren Ölkomponente ganz oder überwiegend aus polaren Estern oder Triglyceridölen besteht, anders: entweder werden (a) trotz einer Phaseninversion keine feinteiligen, blauen Emulsionen gebildet, sondern grobdisperse weiße Emulsionen oder aber es findet (b) im Temperaturbereich bis 100 °C überhaupt keine Phaseninversion statt.

DE-A-38 19 193 beschreibt ein Verfahren zur Herstellung niedrigviskoser O/W-Emulsionen polarer Ölkomponenten, das auf der Methode der Phaseninversionstemperatur (PIT-Methode) beruht. Nach der Lehre dieser Anmeldung werden Phaseninversionstemperaturen unterhalb 100 °C dadurch erreicht, daß neben nichtionischen Emulgatoren weitere Co-Emulgatoren vorhanden sind. Es wurde jedoch gefunden, daß bei Ölen mit einem Dipolmoment oberhalb von 1,96 D nach diesem Verfahren nur grobteilige Dispersionen zugänglich sind. Dies steht in Einklang mit der Publikation von T. Förster, F. Schambil und H. Tesmann, die die Emulgierung nach der PIT-Methode im Hinblick auf selbstemulgierende Tenside und die Polarität des zu emulgierenden Öls untersucht haben (International Journal of Cosmetic Science 1990 [12] 217). Auf Seite 222 führen die Autoren aus, daß das Vorliegen einer Phaseninversion keine Garantie dafür ist, daß feinteilige und lagerstabile Emulsionen erhalten werden.

Aufgabe der vorliegenden Erfindung war es, ein verbessertes Verfahren zur Herstellung feindisperser und langzeitstabiler O/W-Emulsionen auf Basis von Ölmischungen mit einem hohen Anteil an polaren Ölkomponenten zu entwickeln. Insbesondere sollte ein Verfahren bereitgestellt werden, mit dem feindisperse und lagerstabile O/W-Emulsionen auf Basis von Ölen mit einem Dipolmoment oberhalb von 1,96 D hergestellt werden können.

Es wurde nun überraschend gefunden, daß O/W-Emulsionen auf Basis polarer Ölkörper und nichtionischer Emulgatoren dann besonders feinteilig und langzeitstabil sind, wenn man eine Mischung aus polarem Öl, nichtionischem Emulgator und einem speziellen Grenzflächen-Moderator auf eine Temperatur innerhalb oder oberhalb des Phaseninversions-Temperaturbereiches erhitzt - oder die Emulsion bei dieser Temperatur herstellt - und dann die Emulsion auf eine Temperatur unterhalb des Phaseninversion-Temperaturbereiches abkühlt und gegebenenfalls mit Wasser weiterverdünnt.

Gegenstand der Erfindung ist daher ein Verfahren zur Herstellung von Öl-in-Wasser-Emulsionen von polaren Ölkörpern (A), dadurch gekennzeichnet, daß man

(A) 10 bis 90 Gew.-% eines polaren Ölkörpers mit

(B) 0,5 bis 30 Gew.-% eines nichtionischen Emulgators mit einem HLB-Wert von 10 bis 18 und

(C) 0 bis 30 Gew.-% eines Co-Emulgators aus der Gruppe der Fettalkohole mit 12 bis 22 C-Atomen oder der Partialester von Polyolen mit 3 bis 6 C-Atomen mit Fettsäuren mit 12 bis 22 C-Atomen und

(D) 0,01 bis 50 Gew.-% eines Grenzflächen-Moderators, der ausgewählt ist aus der Gruppe der Tocopherole, der Guerbetalkohole mit 16 bis 20 C-Atomen oder eines Steroids mit 1 bis 3 OH-Gruppen

in Gegenwart von 8 bis 85 Gew.-% Wasser bei einer Temperatur oberhalb des Schmelzpunktes des Gemisches aus den Komponenten (A) bis (D) emulgiert und die Emulsion auf eine Temperatur innerhalb oder oberhalb des Phaseninversionstemperaturbereichs erhitzt - oder die Emulsion bei dieser Temperatur herstellt - und dann die Emulsion auf eine Temperatur unterhalb des Phaseninversions-Temperaturbereichs abkühlt und gegebenenfalls mit Wasser weiter verdünnt.

Das erfindungsgemäße Verfahren hat den Vorteil, daß besonders feinteilige, Emulsionen erhalten werden, die eine ausgezeichnete Lagerstabilität aufweisen. Im Vergleich zum bekannten Stand der Technik, z. B. der DE-A-38 19 193, wird darüber hinaus die Phaseninversionstemperatur gesenkt, was in der Praxis wegen der damit verbundenen Energie-Einsparung besonders günstig ist.

Als **polare Ölkörper (A)** eignen sich Mono- und Diester der allgemeinen Formeln (I), (II) und (III)

(I)      $R^1$-COOR$^2$

(II)      $R^2$-OOC-$R^3$-COOR$^2$

(III)      $R^1$-COO-$R^3$-OOC-$R^1$

worin $R^1$ eine Alkylgruppe mit 8 bis 22 C-Atomen und $R^2$ eine Alkylgruppe mit 3 bis 22 C-Atomen und $R^3$ Alkylengruppen mit 2 bis 16 C-Atomen bedeuten und die mindestens 11 und höchstens 40 C-Atome enthalten.

Ölkörper vom Typ der Mono- und Diester der Formeln (I), (II) und (III) sind als kosmetische und pharmazeutische Ölkomponenten sowie als Gleit- und Schmiermittelkomponenten bekannt. Unter den Mono- und Diestern dieser Art kommt den bei Raumtemperatur (20 °C) flüssigen Produkten die größte Bedeutung zu. Als Ölkörper geeignete Monoester (I) sind z.B. die Isopropylester von Fettsäuren mit 12 - 22 C-Atomen, wie z.B. Isopropylmyristat, Isopropylpalmitat, Isopropylstearat, Isopropyloleat. Andere geeignete Monoester sind z.B. n-Butylstearat, n-Hexyllaurat, n-Decyloleat, Isooctylstearat, Isononylpalmitat, Isononyl-isononanoat, 2-Ethylhexyl-palmitat, 2-Ethylhexyl-laurat, 2-Hexyldecyl-stearat, 2-Octyldodecyl-palmitat, Oleyloleat, Oleylerucat, Erucyloleat sowie Ester, die aus technischen aliphatischen Alkoholgemischen und technischen aliphatischen Carbonsäuren erhältlich sind, z.B. Ester aus gesättigten und ungesättigten Fettalkoholen mit 12 - 22 C-Atomen und gesättigten und ungesättigten Fettsäuren mit 12 - 22 C-Atomen, wie sie aus tierischen und pflanzlichen Fetten zugänglich sind. Geeignet sind auch natürlich vorkommende Monoester- bzw. Wachsester-Gemische, wie sie z.B. im Jojobaöl oder im Spermöl vorliegen.

Geeignete Dicarbonsäureester (II) sind z.B. Di-n-butyl-adipat, Di-n-butylsebacat, Di-(2-ethylhexyl)-adipat, Di-(2-hexyldecyl)-succinat und Di-isotridecyl-acelaat. Geeignete Diolester (III) sind z.B. Ethylenglykol-dioleat, Ethylenglykol-di-isotridecanoat, Propylenglykol-di-(2-ethylhexanoat), Propylenglykol-di-isostearat, Propylenglykol-di-pelargonat, Butandiol-di-isostearat und Neopentylglykol-di-caprylat.

Als Ölkörper gut geeignet sind ferner Ester von drei- und mehrwertigen Alkoholen, insbesondere pflanzliche Triglyceride, z.B. Olivenöl, Mandelöl, Erdnußöl, Sonnenblumenöl oder auch die Ester des Pentaerythrits mit z.B. Pelargonsäure oder Ölsäure.

Als Fettsäuretriglyceride können natürliche, pflanzliche Öle, z. B. Olivenöl, Sonnenblumenöl, Sojaöl, Erdnußöl, Rapsöl, Mandelöl, Palmöl, aber auch die flüssigen Anteile des Kokosöls oder des Palmkernöls sowie tierische Öle, wie z. B. Klauenöl, die flüssigen Anteile des Rindertalges oder auch synthetische Triglyceride, wie sie durch Veresterung von Glycerin mit Fettsäuren mit 8 - 22 C-Atomen erhalten werden, z. B. Triglyceride von Caprylsäure-Caprinsäure-Gemischen, Triglyceride aus technischer Ölsäure oder aus Palmitinsäure-Ölsäure-Gemischen.

Bevorzugt eignen sich solche Mono- und Diester und Triglyceride als Ölkomponenten für das erfindungsgemäße Verfahren, die bei Normaltemperatur von 20 °C flüssig sind, es können aber auch höherschmelzende Fette und Ester, die den angegebenen Formeln entsprechen, in solchen Mengen mitverwendet werden, daß die Mischung der Ölkomponenten bei Normaltemperatur flüssig bleibt.

Die Ölkomponente kann auch Kohlenwasserstofföle in untergeordneten Mengen bis zu maximal 25 Gew.-%, bezogen auf die Ölkomponente, enthalten. Geeignete Kohlenwasserstoffe sind vor allem Paraffinöle und synthetisch hergestellte Kohlenwasserstoffe, z. B. flüssige Polyolefine oder definierte Kohlenwasserstoffe, z. B. Alkylcyclohexane, wie z. B. das 1.3-Di-isooctylcyclohexan.

Unter den Ölkörpern (A) verdienen diejenigen besondere Beachtung, die ein Dipolmoment oberhalb von 1,96 D aufweisen, weil bei diesen Ölen die Herstellung feinteiliger statt grobteiliger O/W-Emulsionen durch das erfindungsgemäße Vefahren überhaupt erst möglich wird. Diese Gruppe der Ölkörper (A) kann insbesondere durch ein Dipolmoment im Bereich von 2,0 bis 3,6 D charakterisiert werden. Beispiele dafür sind Decyloleat (2,19 D), Capryl/Caprinsäuretriglycerid (2,78 D) und Mandelöl (3,16 D).

Die polaren Ölkörper (A) werden in den erfindungsgemäßen O/W-Emulsionen in einer Menge von 10 bis 90 Gew.-%, vorzugsweise von 20 bis 60 Gew.-%, eingesetzt.

Als **nichtionische Emulgatoren (B)** geeignete Substanzen sind gekennzeichnet durch eine lipophile, bevorzugt lineare Alkyl- oder Acylgruppe und eine hydrophile, aus niedermolekularen Glycol-, Glucose- und Polyolethern gebildete Gruppe.

Die nichtionischen Emulgatoren (B) werden in den erfindungsgemäßen O/W-Emulsionen in ein Menge von 0,5 bis 30 Gew.-%, vorzugsweise von 3 bis 20 Gew.-%, eingesetzt.

Als nichtionische Emulgatoren (B) eignen sich insbesondere Ethylenoxidanlagerungsprodukte an Fettalkohle mit 16 - 22 C-Atomen. Derartige Produkte sind handelsüblich. Die technischen Produkte stellen Gemische homologer Polyglycolether der Ausgangsfettalkohole dar, deren mittlerer Oxethylierungsgrad der angelagerten Molmenge an Ethylenoxid entspricht. Als Emulgatoren können auch Ethylenoxidanlagerungsprodukte an Partialester aus einem Polyol mit 3 - 6 C-Atomen und Fettsäuren mit 14 - 22 C-Atomen verwendet werden. Solche Produkte werden z.B. durch Ethoxylierung von Fettsäurepartialglyceriden oder von Mono- und Di-Fettsäureestern des Sorbitans, z.B. von Sorbitanmonostearat oder Sorbitansesquioleat hergestellt. Die für das erfindungsgemäß Verfahren geeigneten Emulgatoren sollen einen HLB-Wert von 10 bis 18 aufweisen. Unter dem HLB-Wert (Hydrophil-Lipophil-Balance) soll ein Wert verstanden werden, der errechnet werden kann gemäß

$$HLB = \frac{100 - L}{5}$$

worin L der Gewichtsanteil der lipophilen Gruppen, d. h. der Fettalkyl- oder Fettacylgruppen in Prozent in den Ethylenoxidanlagerungsprodukten ist.

Bevorzugt eignen sich als Emulgatoren (B) Fettalkoholpolyglykolether (B1) der allgemeinen Formel (IV)

$R^1$-(O-CH$_2$-CH$_2$)$_n$-OH     (IV)

in der $R^1$ einen gesättigten oder ungesättigten, geradkettigen oder verzweigten Kohlenwasserstoffrest mit 8 bis 22 C-Atomen, vorzugsweise 12 bis 18 C-Atomen und n eine ganze Zahl von 10 bis 50, vorzugsweise von 10 bis 30, bedeutet, sowie Anlagerungsprodukte von 4 bis 20 Mol Ethylenoxid an ein oder mehrere Fettsäurepartialglyceride (B2).

Unter Fettsäurepartialglyceriden (B2) von gesättigten oder ungesättigten Fettsäuren mit 10 bis 20 C-Atomen sind dabei technische Gemische von Fettsäuremono-, di- und triglyceriden zu verstehen, die durch Veresterung von 1 Mol Glycerin mit 1 bis 2 Mol einer (C$_{10-20}$)-Fettsäure oder durch Umesterung von 1 Mol eines (C$_{10-20}$)-Fettsäuretriglycerids, z.B. von Rindertalg, Schweineschmalz, Palmöl, Sonnenblumenöl oder Sojaöl mit 0,5 bis 2 Mol Glycerin erhalten werden. Handelsüblich sind zwei Typen von Partialglyceriden. Partialglyceride des Typs I enthalten 35 bis 60 % Monoglyceride, 35 bis 50 % Diglyceride und 1 bis 20 % Triglyceride. Partialglyceride des Typs II werden durch Molekulardestillation aus solchen des Typs I hergestellt und enthalten 90 bis 96 % Monoglyceride, 1 bis 5 % Diglyceride und weniger als 1 % Triglyceride (vergl. dazu: a) G.Schuster und W. Adams: Zeitschrift für Lebensmitteltechnologie, 1979, Band 30(6), S. 256-264; b) G.Schuster (Hrsg.) "Emulgatoren für Lebensmittel", Springer-Verlag, 1985). Die erfindungsgemäß verwendeten Fettsäurepartialglyceride sollen 35 bis 96 % Monoglyceride, 1 bis 50 % Diglyceride und 0,1 bis 20 % Triglyceride enthalten.

Bevorzugt geeignet als Emulgatoren sind Anlagerungsprodukte von 8 - 12 Mol Ethylenoxid an gesättigte Fettalkohole mit 16 - 22 C-Atomen. Zur erfindungsgemäßen Emulgierung von Ölkomponenten, die keine unpolaren Kohlenwasserstofföle enthalten, die also aus 50 - 100 Gew.-% Mono- und Diestern der Formeln I, II und III und 0 - 50 Gew.-% Fettsäuretriglyceriden bestehen, eignen sich als Emulgatoren insbesondere Anlagerungsprodukte von 8 - 12 Mol Ethylenoxid an einen gesättigten Fettalkohol mit 18 - 22 C-Atomen.

Zusätzlich zum Emulgator kann in vielen Fällen ein **Co-Emulgator (C)** zur Herstellung der Öl-in-Wasser-Emulsionen nach dem erfindungsgemäßen Verfahren nützlich sein. Als Coemulgatoren sind erfindungsgemäß solche vom Typ der Fettalkohole mit 16 - 22 C-Atomen, z. B. Cetylalkohol, Stearylalkohol, Arachidylalkohol oder Behenylalkohol oder Gemische dieser Alkohole geeignet, wie sie bei der technischen Hydrierung von pflanzlichen und tierischen Fettsäuren mit 16 - 22 C-Atomen oder der entsprechenden Fettsäuremethylester erhalten werden. Weiterhin eignen sich als Coemulgatoren Partialester aus einem Polyol mit 3 - 6 C-Atomen und Fettsäuren mit 14 - 22 C-Atomen. Solche Partialester sind z. B. die Monoglyceride von Palmitin- und/oder Stearinsäure, die Sorbitanmono- und/oder -diester von Myristinsäure, Palmitinsäure, Stearinsäure oder von Mischungen dieser Fettsäuren, die Monoester aus Trimethylolpropan, Erythrit oder Pentaerythrit und gesättigten Fettsäuren mit 14 - 22 C-Atomen. Als Monoester werden auch die technischen Monoester verstanden, die durch Veresterung von 1 Mol Polyol mit 1 Mol Fettsäure erhalten werden und die ein Gemisch aus Monoester, Diester und unverestertem Polyol darstellen.

Besonders gut eignen sich für das erfindungsgemäße Verfahren als Co-Emulgatoren Cetylalkohol, Stearylalkohol oder ein Glycerin, Sorbitan- oder Trimethylolpropan-Monoester einer Fettsäure mit 14 - 22 C-Atomen oder Gemische dieser Stoffe.

Die Co-Emulgatoren (C) werden in den erfindungsgemäßen O/W-Emulsionen in einer Menge von 0 bis 30 Gew.-%, vorzugsweise von 1 bis 20 Gew.-%, eingesetzt.

Der **Grenzflächen-Moderator (D)** wird ausgewählt aus der Gruppe der Tocopherole, der Guerbetalkohole oder eines Steroids mit 1 bis 3 OH-Gruppen. Unter **Tocopherolen** sind Naturstoffe mit Vitamin E-Charakter zu verstehen, die sich vom 2-Methyl-2-(4'.8'.12'-trimethyltridecyl)-chroman-6-ol, dem sogenannten Tocol, ableiten. Die Kennzeichnung erfolgt mit griechischen Buchstaben (vergl. "Römpps Chemie-Lexikon", O.-A.Neumüller (Hrsg.), 7. Aufl., Stuttgart 1977, S. 3615f). Besonders bevorzugt im Sinne der Erfindung ist $\alpha$-Tocopherol, das am häufigsten vorkommende und technische bedeutendste Tocopherol, das vielfach auch als das eigentliche Vitamin E bezeichnet wird. Unter **Guerbetalkoholen** sind spezielle verzweigte Alkohole zu verstehen (vergl. z.B. A.J. O'Lenick Jr., R.E. Bilbo, Soap Cosm. Chem. Spec. 1987 (4) 52). Die erfindungsgemäß einzusetzenden Guerbetalkohole sollen 16 bis 20 C-Atome aufweisen, wie z.B. 2-Hexyldecanol oder 2-Octyldodecanol. Als besonders geeignet hat sich 2-Octyldodecanol erwiesen. Unter **Steroiden** ist eine Gruppe von natürlich auftretenden oder synthetisch gewonnenen Verbindungen zu verstehen, denen das Gerüst des (partiell) hydrierten Cyclopenta[a]phenanthrens zugrunde liegt, vergl. z.B. O.A. Neumüller, Römpps Chemie-Lexikon, 7. Aufl., Stuttgart 1975, S. 3336 ff. Die erfindungsgemäß einzusetzenden Steroide sollen 1 bis 3 OH-Gruppen aufweisen. Besonders geeignet sind die Sterine, bei denen sich am dritten C-Atom des Steroidgerüstes eine OH-Gruppe befindet. Die Sterine treten in allen tierischen und pflanzlichen Zellen auf. Nach ihrem Vorkommen teilt man sie in Zoosterine, z.B. Cholesterin, und Phytosterine, die vorwiegend in höheren Pflanzen vorkommen, auf. Ein besonders geeignetes Steroid ist Cholesterin.

Die Wirkungsweise des Grenzflächen-Moderators beruht wahrscheinlich darauf, daß er wie das zu emulgierende Öl eine hydrophobe Grundstruktur aufweist, diese jedoch durch die sehr polare OH-Gruppe modifiziert ist, die in der Lage ist, in Gegenwart von Wasser, Wasserstoffbrücken auszubilden.

Der Grenzflächen-Moderator (D) wird in den erfindungsgemäßen O/W-Emulsionen in einer Menge von 0,01 bis 50 Gew.-%, vorzugsweise von 0,1 bis 30 Gew.-% und insbesondere von 0,5 bis 15 Gew.-% eingesetzt. Dabei werden besonders gute Ergebnisse im Sinne der Erfindung dann erhalten, wenn das Gewichts-Verhältnis von Ölkörper (A) und Grenzflächen-Moderator (D) im Bereich von 1:0,1 bis 1:1 liegt.

Das erfindungsgemäße Verfahren kann in der Weise durchgeführt werden, daß zunächst die Phaseninversionstemperatur bestimmt wird, indem man eine Probe der auf übliche Weise hergestellten Emulsion unter Verwendung eines Leitfähigkeitsmeßgerätes erhitzt und die Temperatur bestimmt, bei der die Leitfähigkeit stark abnimmt. Die Abnahme der spezifischen Leitfähigkeit der zunächst vorhandenen Öl-in-Wasser-Emulsion nimmt üblicherweise über einen Temperaturbereich von 2 - 8 °C von anfänglich über 1 Millisiemens pro cm (mS/cm) auf Werte unter 0,1 mS/cm ab. Dieser Temperaturbereich wird hier als Phaseninversions-Temperaturbereich (PIT-Bereich) bezeichnet.

Nachdem der PIT-Bereich bekannt ist, kann man das erfindungsgemäße Verfahren entweder in der Weise durchführen, daß man die zunächst wie üblich hergestellte Emulsion nachträglich auf eine Temperatur erhitzt, die innerhalb oder oberhalb des Phaseninversions-Temperaturbereichs liegt, oder in der Weise, daß man bereits bei der Herstellung der Emulsion eine Temperatur wählt, die innerhalb oder oberhalb des Phaseninversions-Temperaturbereichs liegt. Es ist auch möglich ein wasserfreies oder wasserarmes Konzentrat bei der Phaseninversions-Temperatur mit heißem oder kaltem Wasser zu verdünnen (Heiß-Heiß-oder Heiß-Kalt-Verfahren).

Öl-in-Wasser-Emulsionen, wie sie nach dem erfindungsgemäßen Verfahren erhalten werden, finden Anwendung z. B. als Haut- und Körperpflegemittel, als Kühlschmiermittel oder als Textil- und Faserhilfsmittel. Besonders bevorzugt ist das erfindungsgemäße Verfahren zur Herstellung emulsionsförmiger Zubereitungen für die Haut- und Haarbehandlung geeignet.

Die folgenden Beispiele dienen der Erläuterung der Erfindung und sind nicht einschränkend zu verstehen.

**Beispiele**

**1. Allgemeines**

**1.1. Abkürzungen/Konventionen**

In den Kopfzeilen der Tabellen 1 bis 4 sind die Beispiele mit **B1** bis **B10**, die Vergleiche mit **V1** bis **V4** kenntlich gemacht. Die Werte für das Dipolmoment der Ölkörper wurden dabei in der üblichen Einheit Debye angegeben.

In den Tabellen ist:
(a) die Menge der verwendeten Substanzen stets in Gew.-%,
(b) die Art der Emulsion als fein- oder grobteilig und
(c) der PIT-Bereich durch eine untere und obere Temperatur angegeben.

**1.2. Verwendete Substanzen**

**a) polare Ölkörper (A)**

IPM: Isopropylmyristat ("Rilanit$^{(R)}$ IPM"; Fa. Henkel/Düsseldorf)
Cetiol V: Decyloleat ("Cetiol$^{(R)}$ V"; Fa. Henkel/Düsseldorf)
Myritol 318: Capryl/Caprinsäuretriglycerid ("Myritol$^{(R)}$ 318", Fa. Henkel/Düsseldorf)

**b) nichtionische Emulgatoren (B)**

Eumulgin B1: Anlagerungsprodukt von 12 mol Ethylenoxid an 1 mol Cetostearylalkohol; CTFA-Bezeichnung: Ceteareth-12 ("Eumulgin$^{(R)}$ B1; Fa. Henkel/Düsseldorf)

**c) Co-Emulgatoren (C)**

Lanette O: $C_{16/18}$-Fettalkohol; CTFA-Bezeichnung: Cetostearylalkohol ("Lanette$^{(R)}$ O deo"; Fa. Henkel/Düsseldorf)
GMS: Glycerinmonostearat ("Cutina$^{(R)}$ GMS"; Fa. Henkel/Düsseldorf)

**d) Grenzflächen-Moderator (D)**

Vitamin E: Pflanzliches Tocopherol ("Copherol-F-1300"; Fa. Henkel Corp./USA).
Eutanol G: 2-Octyldodecanol ("Eutanol$^{(R)}$ G"; Fa. Henkel/Düsseldorf)

**2. Herstellung und Charakterisierung der Emulsionen**

**2.1. Herstellung der Emulsionen (übliche Arbeitsweise)**

Die Komponenten (A) bis (D) wurden gemischt und auf eine Temperatur oberhalb des Schmelzpunktes der Mischung erwärmt und homogenisiert. Dann wurde die Schmelze unter Rühren in das Wasser, welches auf etwa die gleiche Temperatur erhitzt war, einemulgiert. Die Zusammensetzung der Emulsionen ist den Tabellen 1 bis 4 zu entnehmen.

**2.2. Herstellung der Emulsionen**

Die Emulsionen wurden, wie unter 2.1. beschrieben hergestellt und dann kurzzeitig (ca. 1 Minute) auf 95 °C erhitzt. Dann wurden die Emulsionen rasch, d. h. mit einer Abkühlrate von ca. 2 °C pro Minute, unter Rühren auf Raumtemperatur abgekühlt.

**2.3. Ermittlung der Phaseninversionstemperatur**

Unter Verwendung einer Leitfähigkeitsmeßbrücke (Fa. Radiometer, Kopenhagen) wurde die elektrische Leitfähigkeit der Emulsionen in Abhängigkeit von der Temperatur ermittelt. Zu diesem Zweck wurde die Emulsion zunächst auf + 20 °C abgekühlt. Bei dieser Temperatur zeigten die Emulsionen eine Leitfähigkeit von über 1 Millisiemens pro cm (mS/cm), d. h. sie lagen als Öl-in-Wasser-Emulsionen vor. Durch langsames Erwärmen mit einer Heizrate von ca. 0,5 °C/min, die mit Hilfe eines Temperatur-Programmgebers in Verbindung mit einem Kryostaten gesteuert wurde, wurde ein Leitfähigkeitsdiagramm erstellt. Der Temperaturbereich, innerhalb welchem die Leitfähigkeit auf Werte unterhalb 0,1 mS/cm abfiel, wurde als Phaseninversions-Temperaturbereich notiert.

## 2.4. Beurteilung der Emulsionen

Die erhaltenen O/W-Emulsionen wurden bei 400-facher Vergrößerung im Mikroskop untersucht. Sofern Öltröpfchen erkennbar waren, wurde die Emulsion als "grob" eingestuft; waren dagegen keine Öltröpfchen zu erkennen, so wurde die Emulsion als "fein" bezeichnet. Bei feinteiligen Emulsionen kann dementsprechend von einer Tröpfchengröße unterhalb von 1 um ausgegangen werden.

## 2.5. Ergebnisse

**Beispiele 1 und 2**

Tabelle 1

|  | V1 | B1 | B2 |
|---|---|---|---|
| Lanette O | 4,5 | 4,5 | 4,5 |
| Eumulgin B1 | 6,0 | 6,0 | 6,0 |
| Cetiol V[a)] | 30,0 | 25,0 | 20,0 |
| Vitamin E | - | 5,0 | 10,0 |
| Wasser | 59,5 | 59,5 | 59,5 |
| Emulsion | grob | fein | fein |
| PIT-Bereich (°C) |  |  |  |
| a) untere T. | 87 | 88 | 79 |
| b) obere T. | 90 | 93 | 82 |

[a)] Dipolmoment = 2,19 D

Im Vergleichsversuch V1 wurde nur eine grobteilige Emulsion erhalten. Demgegenüber wurden bei den erfindunsgemäßen Beispielen B1 und B2 feinteilige Emulsionen erhalten; darüber hinaus ist gegenüber V1 die Phaseninversionstemperaturgeringer, das heißt die Herstellung der Emulsion gelingt schon bei niedrigerer Temperatur.

**Beispiele 3 bis 6**

Tabelle 2

| | V2 | B3 | B4 | B5 | B6 |
|---|---|---|---|---|---|
| Lanette O | 4,5 | 4,5 | 4,5 | 4,5 | 4,5 |
| Eumulgin B1 | 6,0 | 6,0 | 6,0 | 6,0 | 6,0 |
| Myristol 318[b] | 30,0 | 25,0 | 20,0 | 25,0 | 20,0 |
| Vitamin E | - | 5,0 | 10,0 | - | - |
| Eutanol G | - | - | - | 5,0 | 10,0 |
| Wasser | 59,5 | 59,5 | 59,5 | 59,5 | 59,5 |
| Emulsion | grob | fein | fein | fein | fein |
| PIT-Bereich (°C) | | | | | |
| a) untere T. | >100[d] | 93 | 84 | 94 | 93 |
| b) obere T. | >100 | 97 | 88 | 99 | 96 |

[b] Dipolmoment = 2,78 D
[d] > 100 bedeutet dabei, daß im Temperaturbereich bis 100 °C keine Phaseninversion beobachtet wurde

Im Vergleichsversuch V2 wurde nur eine grobteilige Emulsion erhalten. Demgegenüber wurden bei den erfindungsgemäßen Beispielen B3 bis B6 feinteilige Emulsionen erhalten; darüber hinaus ist gegenüber V2 die Phaseninversionstemperaturgeringer, das heißt die Herstellung der Emulsion gelingt schon bei niedrigerer Temperatur.

**Beispiele 7 bis 9**

Tabelle 3

| | V3 | B7 | B8 | B9 |
|---|---|---|---|---|
| Lanette O | 4,5 | 4,5 | 4,5 | 4,5 |
| Eumulgin B1 | 6,0 | 6,0 | 6,0 | 6,0 |
| IPM[c] | 30,0 | 25,0 | 22,0 | 15,0 |
| Vitamin E | - | 5,0 | 8,0 | 15,0 |
| Wasser | 59,5 | 59,5 | 59,5 | 59,5 |
| Emulsion | fein | fein | fein | fein |
| PIT-Bereich (°C) | | | | |
| a) untere T. | 86 | 81 | 78 | 68 |
| b) obere T. | 90 | 85 | 81 | 81 |

[c] Dipolmoment = 1,96 D

Der Vergleich V3 zeigt, daß bei Ölen mit einem Dipolmoment bis zu 1,96 D feinteilige Emulsionen auch ohne die Gegenwart eines Grenzflächen-Moderators (D) zugänglich sind. Demgegenüber geht aus den erfindungsgemäßen Beispielen B7 bis B9 klar hervor, daß gegenüber V3 die Phaseninversionstemperatur deutlich gesenkt wird, das heißt die Herstellung der Emulsionen gelingt schon bei niedrigerer Temperatur.

8

**Beispiele 10 und 11**

Tabelle 4

|  | V4 | B10 | B11 |
|---|---|---|---|
| GMS | 4,5 | 4,5 | - |
| Eumulgin B1 | 6,0 | 6,0 | 6,0 |
| IPM[c) | 30,0 | 25,0 | 30,0 |
| Vitamin E | - | 5,0 | 5,0 |
| Wasser | 59,5 | 59,5 | 59,0 |
| Emulsion | fein | fein | fein |
| PIT-Bereich (°C) |  |  |  |
| a) untere T. | 64 | 60 | 90 |
| b) obere T. | 70 | 64 | 95 |

[c) Dipolmoment = 1,96 D

Das Beispiel B10 zeigt gegenüber dem Vergleich V4 dieselbe Verbesserung wie die Beispiele B7 bis B9 gegenüber dem Vergleich V3.

Das Beispiel B11 macht darüber hinaus deutlich, daß die Verwendung eines Co-Emulgators fakultativ und nicht zwingend erforderlich ist

**Patentansprüche**

1.  Verfahren zur Herstellung von Öl-in-Wasser-Emulsionen von polaren Öl-körpern (A), dadurch gekennzeichnet, daß man

    (A) 10 bis 90 Gew.-% eines polaren Ölkörpers mit

    (B) 0,5 bis 30 Gew.-% eines nichtionischen Emulgators mit einem HLB-Wert von 10 bis 18 und

    (C) 0 bis 30 Gew.-% eines Co-Emulgators aus der Gruppe der Fettalkohole mit 12 bis 22 C-Atomen oder der Partialester von Polyolen mit 3 bis 6 C-Atomen mit Fettsäuren mit 12 bis 22 C-Atomen und

    (D) 0,01 bis 50 Gew.-% eines Grenzflächen-Moderators, der ausgewählt ist aus der Gruppe der Tocopherole, der Guerbetalkohole mit 16 bis 20 C-Atomen oder eines Steroids mit 1 bis 3 OH-Gruppen

    in Gegenwart von 8 bis 85 Gew.-% Wasser bei einer Temperatur oberhalb des Schmelzpunktes des Gemisches aus den Komponenten (A) bis (D) emulgiert und die Emulsion auf eine Temperatur innerhalb oder oberhalb des Phaseninversions-Temperaturbereichs erhitzt - oder die Emulsion bei dieser Temperatur herstellt - und dann die Emulsion auf eine Temperatur unterhalb des Phaseninversionstemperaturbereichs abkühlt und gegebenenfalls mit Wasser weiter verdünnt.

2.  Verfahren nach Anspruch 1, dadurch gekennzeichnet, daß man den Grenzflächen-Moderator (D) in einer Menge einsetzt, die 0,1 bis 30 Gew.-% entspricht.

3.  Verfahren nach Anspruch 1 oder 2, dadurch gekennzeichnet, daß man den Grenzflächen-Moderator (D) in einer Menge einsetzt, die 0,5 bis 15 Gew.-% entspricht.

4.  Verfahren nach einem der Ansprüche 1 bis 3, dadurch gekennzeichnet, daß man als Grenzflächen-Moderator (D) $\alpha$-Tocopherol einsetzt.

5.  Verfahren nach einem der Ansprüche 1 bis 3, dadurch gekennzeichnet, daß man als Grenzflächen-Moderator (D) 2-Octyldodecanol einsetzt.

6.  Verfahren nach einem der Ansprüche 1 bis 5, dadurch gekennzeichnet, daß man einen polaren Ölkörper (A) einsetzt, der ein Dipolmoment oberhalb von 1,96 D aufweist.

**7.** Verfahren nach einem der Ansprüche 1 bis 6, dadurch gekennzeichnet, daß man einen polaren Ölkörper (A) einsetzt, der ein Dipolmoment im Bereich von 2,0 bis 3,6 D aufweist.

**8.** Verfahren nach einem der Ansprüche 1 bis 7, dadurch gekennzeichnet, daß man das Gewichts-Verhältnis von Ölkörper (A) und Grenzflächen-Moderator (D) im Bereich von 1:0,1 bis 1:1 einstellt.

**Claims**

**1.** A process for the production of oil-in-water emulsions of polar oil components (A), characterized in that
(A) 10 to 90% by weight of a polar oil component are emulsified with
(B) 0.5 to 30% by weight of a nonionic emulsifier having an HLB value of 10 to 18 and
(C) 0 to 30% by weight of a co-emulsifier from the group of fatty alcohols containing 12 to 22 carbon atoms or partial esters of polyols containing 3 to 6 carbon atoms with fatty acids containing 12 to 22 carbon atoms and
(D) 0.01 to 50% by weight of an interfacial moderator selected from the group of tocopherols, Guerbet alcohols containing 16 to 20 carbon atoms or a steroid containing 1 to 3 OH groups
in the presence of 8 to 85% by weight of water at a temperature above the melting point of the mixture of components (A) to (D) and the emulsion is heated to a temperature within or above the phase inversion temperature range - or the emulsion is prepared at that temperature - and is then cooled to a temperature below the phase inversion temperature range and optionally further diluted with water.

**2.** A process as claimed in claim 1, characterized in that the interfacial moderator (D) is used in a quantity corresponding to 0.1 to 30% by weight.

**3.** A process as claimed in claim 1 or 2, characterized in that the interfacial moderator (D) is used in a quantity corresponding to between 0.5 and 15% by weight.

**4.** A process as claimed in any of claims 1 to 3, characterized in that $\alpha$-tocopherol is used as the interfacial moderator (D).

**5.** A process as claimed in any of claims 1 to 3, characterized in that 2-octyl dodecanol is used as the interfacial moderator (D).

**6.** A process as claimed in any of claims 1 to 5, characterized in that a polar oil component (A) with a dipole moment above 1.96 D is used.

**7.** A process as claimed in any of claims 1 to 6, characterized in that a polar oil component (A) with a dipole moment of 2.0 to 3.6 D is used.

**8.** A process as claimed in any of claims 1 to 7, characterized in that the ratio by weight of the oil component (A) to the interfacial moderator (D) is in the range from 1:0.1 to 1:1.

**Revendications**

**1.** Procédé de préparation d'émulsions d'huile dans l'eau de composants huileux polaires (A), caractérisé en ce que l'on émulsionne
(A) 10 à 90 % en poids d'un corps huileux polaire avec
(B) 0,5 à 30 % en poids d'un émulsifiant non ionique présentant une valeur HLB comprise entre 10 et 18 et
(C) 0 à 30 % en poids d'un co-émulsifiant, appartenant au groupe des alcools gras comportant 12 à 22 atomes de C ou des esters partiels de polyols présentant 3 à 6 atomes de C avec des acides gras comportant 12 à 22 atomes de C et
(D) 0,01 à 50 % en poids d'un modérateur d'interface, sélectionné dans le groupe constitué des tocophérols, des alcools de Guerbet comportant 16 à 20 atomes de C ou d'un stéroïde comportant 1 à 3 groupes OH
en présence de 8 à 85 % en poids d'eau, à une température supérieure au point de fusion du mélange des composants (A) à (D), et en ce que l'on chauffe l'émulsion à une température comprise dans ou au-delà de la plage de température d'inversion de phase - ou en ce que l'on prépare l'émulsion à cette température, la refroidit ensuite à une température inférieure à la plage de température d'inversion de phase et la dilue encore, le cas échéant, avec de l'eau.

2. Procédé selon la revendication 1, caractérisé en ce que l'on met en oeuvre le modérateur d'interface (D) dans une proportion correspondant 0,1 à 30 % en poids.

3. Procédé selon la revendication 1 ou 2, caractérisé en ce que l'on met en oeuvre le modérateur d'interface (D) dans une proportion correspondant à 0,5 à 15 % en poids.

4. Procédé selon une des revendications 1 à 3, caractérisé en ce que l'on met en oeuvre comme modérateur d'interface (D), de l'$\alpha$-tocophérol.

5. Procédé selon une des revendications 1 à 3, caractérisé en ce que l'on met en oeuvre comme modérateur d'interface (D), du 2-octyldodécanol.

6. Procédé selon une des revendications 1 à 5, caractérisé en ce que l'on met en oeuvre un corps polaire (A), qui présente un moment dipolaire supérieur à 1,96 D.

7. Procédé selon une des revendications 1 à 6, caractérisé en ce que l'on met en oeuvre un corps polaire (A), qui présente un moment dipolaire compris dans l'intervalle de 2,0 à 3,6 D.

8. Procédé selon une des revendications 1 à 7, caractérisé en ce que l'on ajuste le rapport pondéral entre le corps huileux (A) et le modérateur d'interface (D) dans l'intervalle de 1:0,1 à 1:1.